# EUROPEAN PATENT SPECIFICATION

(11) **EP 3 968 927 B1**
(45) Date of publication and mention of the grant of the patent: **26.03.2025**
(21) Application number: 19726344.5
(22) Date of filing: 16.05.2019
(51) Int. Cl.: A61F 13/42, H04Q 9/00

(54) **HYGIENE MONITORING DEVICE WITH NARROW DATA LOGGING UNIT**
HYGIENEÜBERWACHUNGSVORRICHTUNG MIT SCHMALER DATENPROTOKOLLIERUNGSEINHEIT
DISPOSITIF DE SURVEILLANCE D'HYGIÈNE AVEC UNITÉ D'ENREGISTREMENT DE DONNÉES RESTREINTE

(43) Date of publication of application: 23.03.2022
(73) Proprietor: Essity Hygiene and Health Aktiebolag, 405 03 Göteborg (SE)
(72) Inventor: AMBRING, Josefin, 417 23 Göteborg (SE)
(74) Representative: Essity Hygiene and Health AB
(86) International application number: PCT/EP2019/062670
(87) International publication number: WO 2020/228960

(56) References cited:
- KR-A- 20120 009 644
- US-A- 5 845 644
- US-A1- 2013 018 340
- US-A1- 2016 080 841

## Description

### Field of the Invention

The present invention relates to a hygiene monitoring device comprising an elongate flexible sensing panel defining a longitudinal axis and a transversal axis and being longer in the longitudinal direction than it is wide in the transverse direction, and a data logger unit attached to, or removably attachable to, the flexible sensing panel, said data logger unit defining a longitudinal axis and a transversal axis, and having a longitudinal length that is longer than its transversal width.

The present invention also relates to a system comprising a hygiene monitoring device and at least one hygiene absorbent article.

### Background

Absorbent articles, such as diapers, absorbent underwear, sanitary products and incontinence shields require periodic replacement, in use, to ensure that the absorbency of the article is not compromised.

In many settings, including domestic settings, institutional settings, healthcare settings and the like, there is a need to monitor the state of an absorbent article provided to a user to ensure that the article contains satisfactory absorbent capacity to fulfil its function.

Conventionally, such monitoring may be performed as self-monitoring by the user, in which the user informs care staff that the capacity of the absorbent article to absorb has diminished, or by periodic checking of the absorbent article by the user or by care staff. However, such processes are labour intensive. Moreover, the information gathered about the personal needs of the user in respect of the needed frequency of replacement of the absorbent article or the needed capacity of the absorbent article is slow to aggregate and is frequently and incompletely collected.

Accordingly, it has been proposed to provide absorbent articles with sensors, which are coupled to data logging electronics. The data logging electronics can determine the absorbent state of the absorbent article, and, for example, can notify a carer when the absorbent state of the absorbent article has reached a predetermined state.

Arrangements have been proposed which use sensing wires embedded in an absorbent core of the absorbent article, such that data logging electronics measure the resistance between the wires to determine the presence of liquid in the absorbent core, and hence the state of the absorbent article.

However, because such sensors are difficult to integrate into the absorbent core of an absorbent article, the sensing capability may be reduced in systems using replaceable logging packages. Moreover, since absorbent articles are often provided in a range of forms and sizes, a logging package which is appropriate for provision to one type or size of absorbent article may perform poorly when used in combination of another type or size of absorbent article.

US5855644 relates to a bowel and bladder training system provides a generally rectangular pad which may have a pocket at one end, with a pair of sensor threads fixed longitudinally in the pad.

Arrangements have also been proposed which use removably attachable sensors to the outer surface of the absorbent product. Such arrangement may be beneficial from a sustainability and recycling point of view as the electronics and sensor wires need not be disposed together with the absorbent article but may instead be reused and/or disposed of separately.

US2013018340 relates to a wetness monitoring system for an absorbent article, the wetness monitoring system including a signalling device including an alarm to indicate that the absorbent article has reached an insult limit. The signalling device operates with a sensor array which is disposed on the outermost surface of the absorbent article outer cover.

However, such externally positioned sensors have shown disadvantages in reducing the wearing comfort of the absorbent articles, as well as being prone to fall off the absorbent article during use, leading to incomplete or misleading acquisition of information.

Accordingly, there is need for means of measuring and monitoring the absorbent status of absorbent articles which overcomes at least some of the drawbacks associated with prior arrangements.

### Summary of the Invention

According to a first aspect of the present invention, there is provided a hygiene monitoring device according to claim 1 comprising an elongate flexible sensing panel and a data logger unit. The elongate flexible sensing panel defines a longitudinal axis and a transversal axis and is longer in the longitudinal direction than it is wide in the transverse direction. The data logger unit is attached to, or is removably attachable to, the flexible sensing panel and defines a longitudinal axis and a transversal axis and has a longitudinal length that is longer than its transversal width. When the data logger unit is attached to the sensing panel, the longitudinal axis of the flexible sensing panel is substantially aligned along the longitudinal axis of the data logger unit. The flexible sensing panel comprises a flexible substrate which carries at least a first sensing element. A fastening material is disposed at a first surface of the flexible sensing panel and the data logger unit is disposed on the opposing, second surface of the flexible sensing panel.

The transverse width of the data logger unit may be at most 160, 150, 140, 130, 120 or 110 % of the maximum transverse width of the flexible sensing panel within the longitudinal extent of the flexible sensing panel covered by the data logger unit.

The transverse width of the data logger unit may be at least 80, 90 or 100 % of the transverse width of the flexible sensing panel within the longitudinal extent of the flexible sensing panel covered by the data logger unit.

The ratio of the transverse width of the flexible sensing panel, within the longitudinal extent of the flexible sensing panel covered by the data logger unit, to the longitudinal length of the sensing panel is from 0.025 to 0.1.

The transverse width of the flexible sensing panel, within the longitudinal extent of the flexible sensing panel covered by the data logger unit, may be from 20 or 22 to 30 or 28 mm, such as about 25 mm.

The longitudinal length of the flexible sensing panel is be from 200, 300, 400 or 500, to 700, 600, 500 or 400 mm, such as from 200 to 400 mm or from 500 to 700 mm.

Over at least 60, 70, 80, 90 or 95% of its longitudinal extension, the flexible sensing panel may have substantially straight and parallel longitudinally extending side edges.

The ratio of the transverse width to the longitudinal length of the data logger unit may be from 0.3 to 0.8.

The transverse width of the data logger unit may be from 20, 25 or 30 to 45 or 40 mm, such as about 35 mm.

The longitudinal length of the data logger unit may be from 30, 35, 40, 45 or 50 to 70, 65, 60 or 55 mm, such as about 50 to 55 mm.

The data logger unit may be removably attachable to the sensing panel, and the thickness of the data logger unit, in the direction orthogonal to the longitudinal axis and the transversal axis of the data logger unit, may be from 2, 5, 8 or 10 to 20 or 15 mm, such as about 13 mm.

The hygiene monitoring device is adapted for being removably attached to a garment facing side of an absorbent hygiene product by means of the fastening material. The absorbent hygiene product defines a body facing side, intended to face the body of the wearer during use, and an opposing garment facing side, intended to face away from the wearer during use.

The absorbent hygiene product may be a diaper such as a pant-type diaper, an open-type diaper, a belt-type diaper, or an incontinence pad or a sanitary napkin.

The hygiene monitoring device may be adapted to detect the presence of body fluids absorbed by the absorbent hygiene product.

When the data logger unit is attached to the sensing panel, it may be electrically connected to the at least one sensing element.

A sensing element according to the present disclosure may comprise a first sensing plate and a second sensing plate plates and the hygiene monitoring device may be configured to measure the impedance between said first and second sensing plates.

The fastening material may comprise a hook material for removably securing the sensing panel to a fibrous nonwoven material at the garment facing side of the absorbent hygiene product (20).

The fastening material may cover at least 90%, such as from 90 to 95 to 100 or 99% of the first surface of the sensing panel.

The fastening material may form a continuous area on the first surface of the sensing panel.

When attached to the flexible sensor panel, the longitudinal center of the data logger unit may be positioned closer to a longitudinally outermost edge of the sensor panel than to the longitudinal center of the flexible sensor panel.

The flexible sensing panel max extend longitudinally beyond both longitudinal outermost edges of the data logger unit.

The distance from the longitudinally outermost edge of the data logger unit to the closest one of the longitudinally outermost edges of the flexible sensing panel may be at least 10 mm, such as from 10, 20 or 30 to 60, 50 or 40 mm.

When attached to the flexible sensor panel the transversal center of the data logger unit may be substantially super positioned on the transversal center of the flexible sensing panel.

In a device according to the present disclosure, a panel-side logger attachment means may be arranged on the second surface on the flexible sensing panel and a logger-side logger attachment means may be arranged on the data logger unit. The panel-side logger attachment means and the logger-side logger attachment means are adapted to removably attach the data logger unit to the flexible sensing panel and to electrically connect the data logger unit with the at least one sensing element.

When attached to the flexible sensor panel, the data logger unit may abut the second surface of the flexible sensing panel.

The present disclosure also relates to a system comprising a hygiene monitoring device as disclosed herein, and at least one absorbent hygiene article.

In such system, the hygiene monitoring device may be is removably secured or securable on a garment facing side of the absorbent hygiene article.

### Brief Description of the Drawings

For a better understanding of the invention, and to show how the same may be carried into effect, reference will now be made, by way of example, to the accompanying drawings, in which:
Figure 1 is a schematic top view of a flexible sensing panel
Figure 2 is a schematic top view of a data logger unit
Figure 3 is a schematic top view of a hygiene monitoring device with a data logger arranged on a flexible sensing panel
Figure 4 is a schematic side-view of a hygiene monitoring device with a data logger arranged on a flexible sensing panel.
Figure 5 shows a schematic drawing of a hygiene monitoring device secured on the garment facing side of an absorbent hygiene product.

### Detailed Description

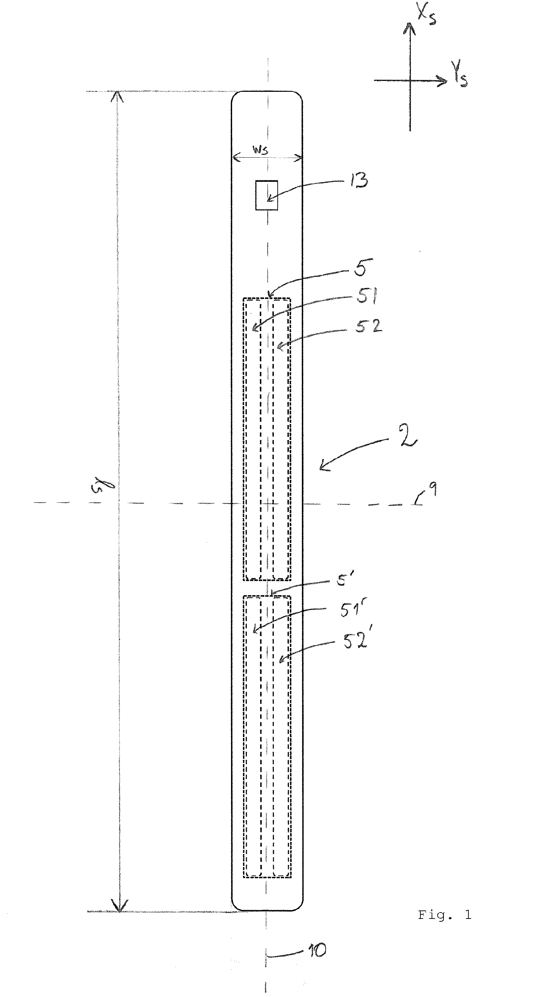

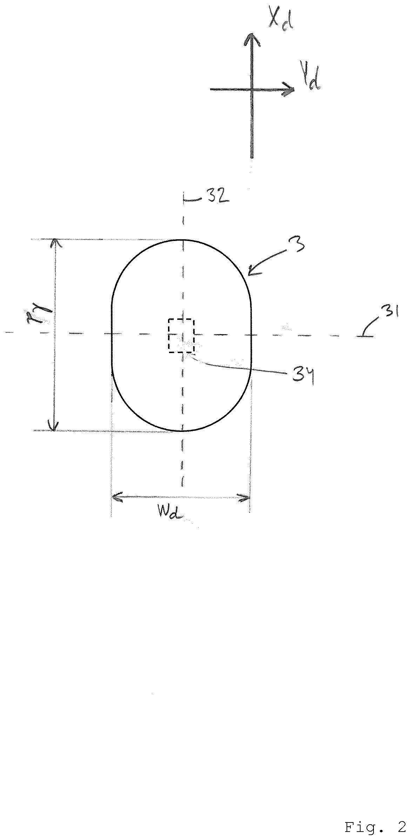

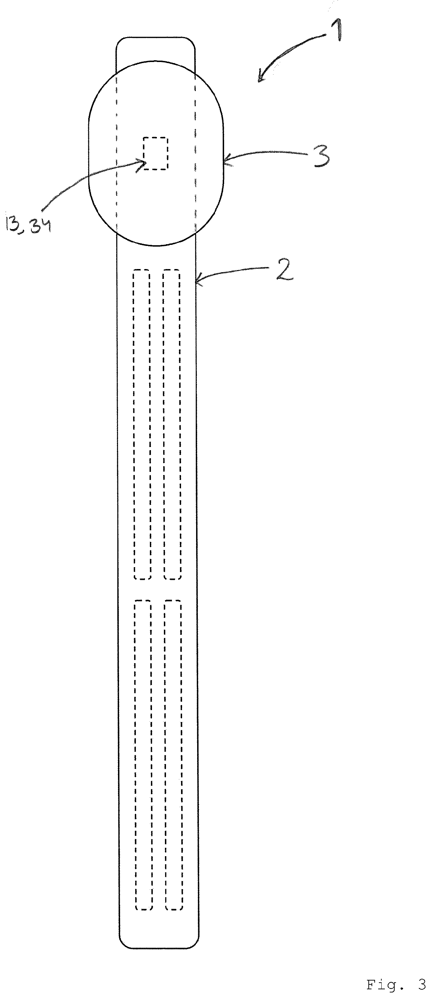

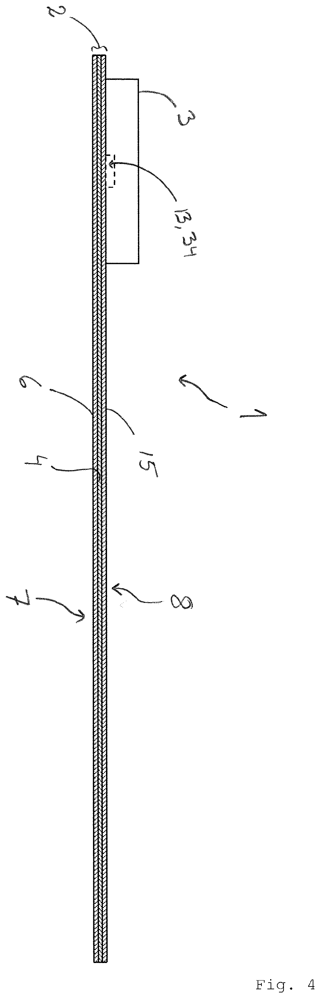

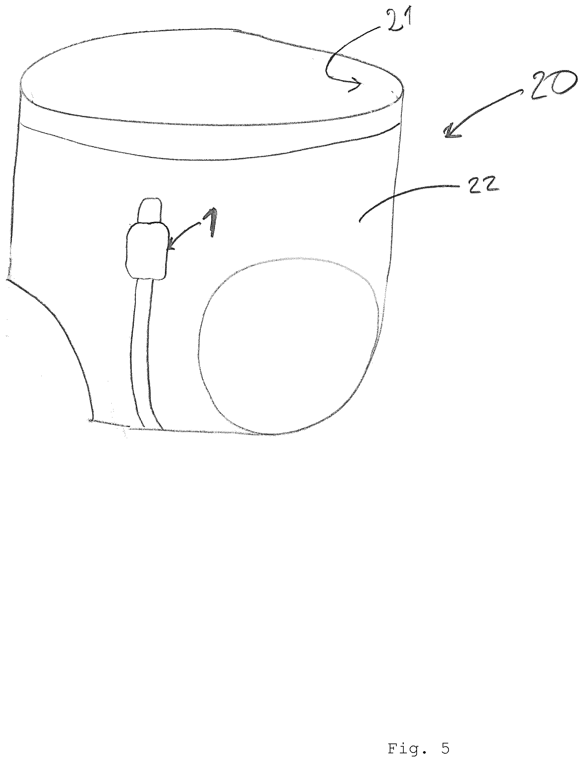

A hygiene monitoring device 1 of the present disclosure comprises a flexible sensing panel 2 and a data logger unit 3 attached to, or removably attachable to the flexible sensing panel 2. Figures 1 and 2 shows the flexible sensing panel 2 and the data logger unit 3 in isolation from each other, while figures 3 and 4 show the hygiene monitoring device 1 with the flexible sensing panel 2 and the data logger unit attached to each other, and figure 5 shows a hygiene monitoring device secured on the garment facing side 22 of an absorbent hygiene product 20.

As shown in figure 1, flexible sensing panel 2 has a length along the longitudinal axis Xs longer that the width along the transverse axis Ys, giving it a generally elongate shape. While the sensing panel in figure 1 has a generally rectangular shape, other shapes of the flexible sensing panel are also possible within the scope of the present disclosure.

The length lₛ of the flexible sensing panel 2 will depend on the size of the absorbent product on which it is to be secured, but may vary from about 200 to about 700 mm.

The transversal width wₛ is measured in proximity to the sensing-panel side logger attachment means 13 to represent the maximum width of the flexible sensing panel 2 in the longitudinal extent thereof that will be covered by a data logger unit 3, when the data logger unit 3 is attached to the flexible sensing panel, and may be from 20 or 22 to 30 or 28 mm, such as about 25 mm. The ratio of the transverse width wₛ to the longitudinal length lₛ of the sensing panel may be from 0.025 to 0.1

Towards one of the longitudinal edges of the flexible sensing panel 2 a panel-side logger attachment means 13 is disposed for connecting the flexible sensing panel 2 to a data logger unit 3, which is to be described below.

The flexible sensing panel 2 is generally made from one or more flexible, easily bendable materials, but may comprise rigid components, such as the panel-side logger attachment means 13.

As is shown in figure 4, the flexible sensing panel 2 comprises a flexible substrate 4 such as flexible printed circuit board (flex-PCB). The flexible substrate 4 carries a first sensing element 5 and a second sensing element 5' that are adapted to be used for detecting the presence of body liquids absorbed in a hygiene absorbent product to which the hygiene monitoring device 1 is secured. The sensing elements 5, 5' may be printed on the flexible substrate or otherwise arranged thereon. The sensing elements 5, 5' are electrically connected by conductive leads (not shown) to the panel-side logger attachment means 13.

As can be viewed in figure 4, on the side of the flexible substrate 4 opposing that of the panel-side logger attachment means 13, a fastener material 6 is disposed. The fastener material 6 is adapted to removably secure the flexible sensing panel 2 to an absorbent hygiene product. This may for example be obtained by means of hooks for fastening to a loop material, such as a nonwoven, on the absorbent hygiene product, or by means of an adhesive for fastening to plastic film surface on the absorbent hygiene product. Hook materials suitable for use include hook tapes obtainable from commercial sources, such as Velcro and 3M. The fastening material 6 advantageously covers essentially the full area, or at least 95, or at least 90% of that side of the flexible substrate.

On the side of the flexible substrate 4 opposite to the fastening material, i.e. on the side of the panel-side logger attachment means 13, a protective cover layer 15, such as a tape or a coating layer, may optionally be arranged to protect the flexible substrate 4 and/or to provide a suitable surface structure. Figure 2 shows an exemplary configuration of a data logger unit 3 being one component of a hygiene monitoring device 1 shown I figures 3 and 4. The data monitoring unit 3 is adapted to be removably attachable to the flexible sensing panel 2.

The data logger unit 3 has a longitudinal length l_{d} that is longer than its transverse width w_{d}, to obtain an elongate shape, for example with an oblong-like shape

The data logger unit 3 comprises a logger-side logger attachment means 34 that is adapted to interact with the panel-side logger attachment means 13.

The logger-side logger attachment means 34 and the panel-side logger attachment means are arranged such that when the data logger unit 3 is attached to the flexible sensing panel, the longitudinal axis Xd of the logger unit 3 is arranged substantially along the longitudinal axis Xs of the flexible sensing panel 2.

The width w_{d} of the data logger unit 3 may be up to 160% of the width wₛ of the flexible sensing panel. Therefore, by allowing the data logger unit 3 to be relatively narrow, it will not significantly reduce the wearing comfort of an absorbent product equipped with a hygiene monitoring device 1 of the present disclosure, as the data logger unit 3 will not add a significant amount of comfort reduction.

The width w_{d} of the data logger unit 3 may also be at least 80% of the of the width wₛ of the flexible sensing panel. Therefore, by allowing the data logger unit 3 to be relatively close in width to the underlying flexible sensing strip, it will be able to be clearly recognizable and easy to grab when it is to be removed from the flexible sensing strip 2.

The ratio of the transverse width w_{d} to the longitudinal length l_{d} of the data logger unit 3 may be from 0.3 to 0.8.

The transverse width w_{d} of the data logger unit may be from 20, 25 or 30 to 45 or 40 mm, such as about 35 mm.

The longitudinal length l_{d} of the data logger unit may be from 30, 35, 40, 45 or 50 to 70, 65, 60 or 55 mm, such as about 50 to 55 mm.

The thickness of the data logger unit 3, in the direction orthogonal to the longitudinal axis Xd and the transversal axis Yd of the data logger unit may preferably be less than 20 mm, such as from 2, 5 or 8 to 20, 15 or 10 mm. A low thickness reduces the discomfort for the user during use of the absorbent product 20 when a hygiene monitoring device 1 is secured thereto.

The longitudinal center 31 of the data logger unit 3, represented by a transversal line dividing the data logger unit 3 in two equally long portions, may be positioned closer to a longitudinally outermost edge of the flexible sensing panel 2 than to the longitudinal center 9, represented by a transversal line dividing the data logger unit in two equally long portions of the flexible sensor panel. This positions the data logger unit 3 fairly close to one of the longitudinal edges of the flexible sensing panel 2 and allows for a low impact on the wearer comfort.

The distance from the longitudinally outermost edge of the data logger unit 3 to the closest one of the longitudinally outermost edges of the flexible sensing panel 2 may be at least 10 mm.

This allows for the formation grip tab portion of the flexible sensing panel allowing easy securing and removal of the hygiene monitoring device to an absorbent article.

The transversal center 32 of the data logger unit 3 may, as shown in figure 3, be substantially super positioned on the transversal center 10 of the flexible sensing panel 2a.

When the data logger unit 3 is attached to the flexible sensing panel 2 by means of the logger-side logger attachment means 34 and the panel-side logger attachment means 13, the data logger unit 3 may abut the second surface 8 of the flexible sensing panel 2.

When, as is shown in figures 3 and 4, the data logger unit 3 is attached to the flexible sensing panel 2 by means of the logger-side logger attachment means 34 and the panel-side logger attachment means 13 a physical attachment is obtained while also electrically connecting the electronic circuitry housed in the data logger unit with the sensing elements 5, 5' on the flexible sensing panel 2.

The logger-side logger attachment means 34 and the panel-side logger attachment means 13 may be any connector combination as known to those in the art, such as, mezzanine connectors, plug- and-socket connectors, registered jack or modular connectors, pogo-pin connectors, tip-ring-sleeve connectors, D-subminiature connectors, DIN connectors, or other terminal or connector types as known in the art. Where such connectors are gendered, either of the male part or the female part may be provided to data logger unit 3 and the other of the male part or the female part may be provided to sensing panel 2. Such terminal arrangements may also have a function to retain data logger unit 3 and sensing panel 2 in physical engagement as well as electrical connection, especially when locking or otherwise secured variants of such connectors are provided. However, the electrical connection and physical attachment may be obtained by two or more attachment means interacting to form the attachment means 13, 34.

When the electronic circuitry housed in the data logger unit 3 is electrically connected to the sensing elements 5, the hygiene monitoring device 1 is operable and capable of detecting the presence of, and optionally the quantity of, body liquids absorbed in an absorbent hygiene product which the hygiene monitoring device 1 is secured.

The electronic circuitry may comprise a power source, such as a cell battery or a rechargeable battery, sensor-driving electronics for driving the sensing elements 5, 5' and a transmitter for transmitting data such as sensor values and optionally other information to an external device, such as a computer or a hand-held device. The transmitter preferably transmits data by means of a wireless protocol, such as Zigbee, WiFi or Bluetooth. The electronic circuitry may also comprise a receiver for receiving data from an external device.

In the exemplary configuration shown in figure 1, each sensing element comprises a first sensing plate 51, 51' and a second sensing plate 52, 52'. The detection of body liquids involves measuring the impedance between the first sensing plate 51, 51' and the second sensing plate 52, 52' and detecting a change in the impedance value over time. However, the present disclosure is not limited to this specific measuring technique, and may rely on other types of sensors, such as sensors measuring relative humidity, VOC (volatile organic compounds) and/or other gasses, temperature, conductivity etc. However, in embodiments, such as in the case of the impedance measurements, the detection of body liquids may advantageously be performed without requiring direct contact between the sensing panel and the body liquids, especially without requiring galvanic contact between the sensing elements 5, 5' and the body liquid to be detected.

Figure 5 shows an exemplary configuration of a hygiene monitoring device 1 secured on the outside of an absorbent hygiene product 20, defining an inner, body facing side 21 and an outer garment facing side 21. The absorbent hygiene product 20 typically comprises an absorbent core to absorb body fluids such as urine, faces and/or menstrual fluid discharged from the wearer while wearing the absorbent hygiene product 20. The absorbent core is typically arranged between a liquid permeable top-sheet and a substantially liquid impermeable back-sheet as is conventional in the art.

In figure 5, the hygiene absorbent product 20 is shown as a pant-type diaper, however any type of hygiene absorbent product configured to be worn in the crotch area of a wearer may be used with a hygiene monitoring device 1 of the present disclosure, such as open-type diapers, belt-type diapers, pant-type diapers, incontinence shields (pads) or sanitary napkins.

The garment facing side 22 of the hygiene absorbent product 20 may have a surface of fibrous non-woven material, to which a hook-type fastening material 6 of the sensing panel 2 may readily adhere in order to releasably secure the hygiene monitoring device 1 on the hygiene absorbent article 20. The garment facing side 22 of the hygiene absorbent product 20 may alternatively have a plastic film surface, and the fastening material 6 may be an adhesive, such as a pressure sensitive adhesive, in order to releasably secure the hygiene monitoring device 1 on the hygiene absorbent article 20.

In figure 5, the hygiene monitoring device 1 is secured on the hygiene absorbent product 20 with the data logger unit 2 towards a front waist region of the hygiene absorbent product 20, and the flexible sensing panel 2 extending towards the rear waist region of the hygiene absorbent product 20. Alternative positions of the hygiene monitoring device are also possible, such as, but not limited to, with the data logger unit arranged towards a rear waist region of the hygiene absorbent product and the flexible sensing panel 2 extending towards the front waist region of the hygiene absorbent product.

By arranging the data logger unit 3 with its longitudinal axis Xd along the longitudinal axis Xs of the flexible sensing panel 2 the area of the data logger unit 3 is distributed over a larger area of the flexible sensing panel 3. When the hygiene monitoring device is secured on the garment facing side 22 of the hygiene absorbent product 20, the device of the present disclosure therefore reduces the risk of the hygiene monitoring device to fall of the absorbent hygiene product, as the weight of the data logger unit is distributed over a larger area of the fastening material 6.

In another aspect, the present disclosure relates to a system comprising a hygiene monitoring device 1 as disclosed herein, and at least one absorbent hygiene article 20.

In such system, the hygiene monitoring device 1 may be removably secured on a garment facing side 22 of the absorbent hygiene article 20, or it may be provided as a separate entity from the absorbent hygiene article 20. For example, a hygiene monitoring device 1 may be provided in a package containing one or more absorbent hygiene articles 20. In another alternative, a data logger unit 3, one or more sensing panels 2 and one or more absorbent articles 20 may be provided as a system.

## Claims

1. A hygiene monitoring device (1), comprising an elongate flexible sensing panel (2) defining a longitudinal axis (Xₛ) and a transversal axis (Ys) and being longer in the longitudinal direction than it is wide in the transverse direction, and a data logger unit (3) attached to, or removably attachable to, the flexible sensing panel (2), said data logger unit (3) defining a longitudinal axis (X_{d}) and a transversal axis (Y_{d}), and having a longitudinal length (l_{d}) that is longer than its transversal width (w_{d}), and, when the data logger unit (3) is attached to the sensing panel (2), the longitudinal axis (Xₛ) of the flexible sensing panel (2) is aligned along the longitudinal axis (X_{d}) of the data logger unit, wherein the flexible sensing panel (2) comprises a flexible substrate (4) which carries at least a first sensing element (5), wherein a fastening material (6) is disposed at a first surface (7) of the flexible sensing panel (2) and wherein said data logger unit (3) is disposed on the opposing, second surface (8) of the flexible sensing panel (2), said device (1) being adapted for being removably attached to a garment facing side (22) of an absorbent hygiene product (20), by means of said fastening material (6), said absorbent hygiene product (20) defining a body facing side (21), intended to face the body of the wearer during use, and an opposing garment facing side (22), intended to face away from the wearer during use,
wherein said flexible substrate (4) is a flexible printed circuit boards, and said at least first sensing element (5) is electrically connected by conductive leads to a panel-side logger attachment means (13),
wherein the ratio of the transverse width (wₛ), within the longitudinal extent (14) of the flexible sensing panel (2) covered by the data logger unit (3), to the longitudinal length of the sensing panel is from 0.025 to 0.1,
wherein the transverse width (ws) of the flexible sensing panel (2), within the longitudinal extent (14) of the flexible sensing panel (2) covered by the data logger unit (3), is from 20 to 30 mm, and
wherein the longitudinal length (lₛ) of the flexible sensing panel (2) is from 200 to 700 mm.

2. The device according to claim 1, wherein the transverse width (w_{d}) of the data logger unit (4) is no more than 160 % of the maximum transverse width (wₛ) of the flexible sensing panel within the longitudinal extent (14) of the flexible sensing panel (2) covered by the data logger unit (3) and/or wherein the transverse width (wd) of the data logger unit (3) is at least 80 % of the transverse width (wₛ) of the flexible sensing panel (2) within the longitudinal extent (14) of the flexible sensing panel (2) covered by the data logger unit (3).

3. The device according to any one of the preceding claims, wherein the flexible sensing panel (2), over at least 60 % of its longitudinal extension, has substantially straight and parallel longitudinally extending side edges.

4. The device according to any one of the preceding claims, wherein the ratio transverse width (w_{d}) / longitudinal length (l_{d}) of the data logger unit is from 0.3 to 0.8

5. The device according to any one of the preceding claims, wherein the transverse width (w_{d}) of the data logger unit (3) is from 20 to 45 mm, and/or wherein the longitudinal length (l_{d}) of the data logger unit (3) is from 30 to 70 mm.

6. The device according to any one of the preceding claims, wherein the data logger unit (3) is removably attachable to the flexible sensing panel (2), and the thickness of the data logger unit, in the direction orthogonal to the longitudinal axis (Xd) and the transversal axis (Yd) of the data logger unit (3), is from 2 to 20 mm.

7. The device according to any one of the preceding claims, wherein absorbent hygiene product (20) is a diaper, such as a pant-type diaper, an open-type diaper, a belt-type diaper, or an incontinence pad or a sanitary napkin.

8. The device according to any one of the preceding claims, adapted to detect the presence of body fluids absorbed by the absorbent hygiene product.

9. The device according to any one of the preceding claims, wherein the data logger unit (3), when attached to the sensing panel (2), is electrically connected to the at least one sensing element (5), preferably wherein said sensing element (5) comprises a first sensing plate (51) and a second sensing plate (52) plates and the device is configured to measure the impedance between said first and second sensing plates.

10. The device according to any one of the preceding claims, wherein the flexible sensing panel (2) extends longitudinally beyond both longitudinal edges of the data logger unit (3).

11. The device according to any one of the preceding claims, wherein the distance from the longitudinally outermost edge of the data logger unit (3) to the closest one of the longitudinally outermost edges of the flexible sensing panel (2) is at least 10 mm.

12. The device according to any one of the preceding claims, wherein a panel-side logger attachment means (13) is arranged on the second surface (8) on the flexible sensing panel (2), where a logger-side logger attachment means (34) is arranged on the data logger unit (3), and wherein the panel-side logger attachment means (13) and the logger-side logger attachment means (34) are adapted to removably attach the data logger unit (3) to the flexible sensing panel (2) and to electrically connect the data logger unit (2) with the at least one sensing element (5).

13. A system comprising a hygiene monitoring device (1) as defined in anyone of the claims 1 to 12 and at least one absorbent hygiene article (20), preferably wherein said hygiene monitoring device (1) is removably secured on a garment facing side (22) of the absorbent hygiene article (20).

## Patentansprüche

1. Hygieneüberwachungsvorrichtung (1), die ein längliches flexibles Sensorfeld (2), das eine Längsachse (Xₛ) und eine Querachse (Ys) definiert und in der Längsrichtung länger ist als es in der Querrichtung breit ist, und eine Datenprotokollierereinheit (3) umfasst, die an dem flexiblen Sensorfeld (2) angebracht ist oder abnehmbar an diesem angebracht werden kann, wobei die Datenprotokollierereinheit (3) eine Längsachse (X_{d}) und eine Querachse (Y_{d}) definiert und eine Längslänge (I_{d}) aufweist, die länger ist als ihre Querbreite (w_{d}), und wenn die Datenprotokollierereinheit (3) am Sensorfeld (2) angebracht ist, die Längsachse (Xₛ) des flexiblen Sensorfeldes (2) entlang der Längsachse (X_{d}) der Datenprotokollierereinheit ausgerichtet ist, wobei das flexible Sensorfeld (2) ein flexibles Substrat (4) umfasst, das mindestens ein erstes Sensorelement (5) trägt, wobei ein Befestigungsmaterial (6) an einer ersten Oberfläche (7) des flexiblen Sensorfeldes (2) angeordnet ist und wobei die Datenprotokollierereinheit (3) auf der gegenüberliegenden, zweiten Oberfläche (8) des flexiblen Sensorfeldes (2) angeordnet ist, wobei die Vorrichtung (1) dafür geeignet ist, mittels des Befestigungsmaterials (6) abnehmbar an einer der Kleidung zugewandten Seite (22) eines saugfähigen Hygieneprodukts (20) angebracht zu werden, wobei das saugfähige Hygieneprodukt (20) eine dem Körper zugewandte Seite (21), die dazu gedacht ist, während des Gebrauchs dem Körper des Trägers zugewandt zu sein, und eine gegenüberliegende, der Kleidung zugewandte Seite (22), die dazu gedacht ist, während des Gebrauchs vom Träger abgewandt zu sein, definiert,
wobei das flexible Substrat (4) eine flexible Leiterplatte ist und das mindestens erste Sensorelement (5) durch leitfähige Leitungen elektrisch mit einem feldseitigen Protokollierer-Anbringungsmittel (13) verbunden ist,
wobei das Verhältnis der Querbreite (wₛ) innerhalb der von der Datenprotokollierereinheit (3) abgedeckten Längserstreckung (14) des flexiblen Sensorfeldes (2) zur Längslänge des Sensorfeldes 0,025 bis 0,1 beträgt,
wobei die Querbreite (ws) des flexiblen Sensorfeldes (2) innerhalb der von der Datenprotokollierereinheit (3) abgedeckten Längserstreckung (14) des flexiblen Sensorfeldes (2) 20 bis 30 mm beträgt, und wobei die Längslänge (Is) des flexiblen Sensorfeldes (2) 200 bis 700 mm beträgt.

2. Vorrichtung nach Anspruch 1, wobei die Querbreite (w_{d}) der Datenprotokollierereinheit (4) innerhalb der von der Datenprotokollierereinheit (3) abgedeckten Längserstreckung (14) des flexiblen Sensorfeldes (2) nicht mehr als 160 % der maximalen Querbreite (wₛ) des flexiblen Sensorfeldes beträgt und/oder wobei die Querbreite (wd) der Datenprotokollierereinheit (3) innerhalb der von der Datenprotokollierereinheit (3) abgedeckten Längserstreckung (14) des flexiblen Sensorfeldes (2) mindestens 80 % der Querbreite (wₛ) des flexiblen Sensorfeldes (2) beträgt.

3. Vorrichtung nach einem der vorstehenden Ansprüche, wobei das flexible Sensorfeld (2) über mindestens 60 % seiner Längserstreckung im Wesentlichen gerade und sich in Längsrichtung parallel erstreckende Seitenkanten aufweist.

4. Vorrichtung nach einem der vorstehenden Ansprüche, wobei das Verhältnis Querbreite (w_{d}) / Längslänge (I_{d}) der Datenprotokollierereinheit 0,3 bis 0,8 beträgt.

5. Vorrichtung nach einem der vorstehenden Ansprüche, wobei die Querbreite (w_{d}) der Datenprotokollierereinheit (3) 20 bis 45 mm beträgt und/oder wobei die Längslänge (I_{d}) der Datenprotokollierereinheit (3) 30 bis 70 mm beträgt.

6. Vorrichtung nach einem der vorstehenden Ansprüche, wobei die Datenprotokollierereinheit (3) abnehmbar am flexiblen Sensorfeld (2) angebracht werden kann und die Dicke der Datenprotokollierereinheit in der Richtung orthogonal zur Längsachse (Xd) und der Querachse (Yd) der Datenprotokollierereinheit (3) 2 bis 20 mm beträgt.

7. Vorrichtung nach einem der vorstehenden Ansprüche, wobei das saugfähige Hygieneprodukt (20) eine Windel, wie etwa eine Höschenwindel, eine offene Windel, eine Gürtelwindel oder eine Inkontinenzeinlage oder eine Hygienevorlage ist.

8. Vorrichtung nach einem der vorstehenden Ansprüche, die dazu geeignet ist, das Vorhandensein von Körperflüssigkeiten, die vom saugfähigen Hygieneprodukt aufgesaugt wurden, zu erkennen.

9. Vorrichtung nach einem der vorstehenden Ansprüche, wobei die Datenprotokollierereinheit (3), wenn sie am Sensorfeld (2) angebracht ist, elektrisch mit dem mindestens einen Sensorelement (5) verbunden ist, bevorzugt wobei das Sensorelement (5) eine erste Sensorplatte (51) und eine zweite Sensorplatte (52) umfasst und die Vorrichtung so konfiguriert ist, dass sie die Impedanz zwischen der ersten und der zweiten Sensorplatte misst.

10. Vorrichtung nach einem der vorstehenden Ansprüche, wobei sich das flexible Sensorfeld (2) in Längsrichtung über beide Längskanten der Datenprotokollierereinheit (3) hinaus erstreckt.

11. Vorrichtung nach einem der vorstehenden Ansprüche, wobei der Abstand von der in Längsrichtung äußersten Kante der Datenprotokollierereinheit (3) zur nächstgelegenen der in Längsrichtung äußersten Kanten des flexiblen Sensorfeldes (2) mindestens 10 mm beträgt.

12. Vorrichtung nach einem der vorstehenden Ansprüche, wobei ein feldseitiges Protokollierer-Anbringungsmittel (13) auf der zweiten Oberfläche (8) auf dem flexiblen Sensorfeld (2) angeordnet ist, wobei ein protokolliererseitiges Protokollierer-Anbringungsmittel (34) auf der Datenprotokollierereinheit (3) angeordnet ist, und wobei das feldseitige Protokollierer-Anbringungsmittel (13) und das protokolliererseitige Protokollierer-Anbringungsmittel (34) dazu geeignet sind, die Datenprotokollierereinheit (3) abnehmbar am flexiblen Sensorfeld (2) anzubringen und die Datenprotokollierereinheit (2) elektrisch mit dem mindestens einen Sensorelement (5) zu verbinden.

13. System, das eine Hygieneüberwachungsvorrichtung (1) wie in einem der Ansprüche 1 bis 12 definiert und mindestens einen saugfähigen Hygieneartikel (20) umfasst, bevorzugt wobei die Hygieneüberwachungsvorrichtung (1) abnehmbar auf einer der Kleidung zugewandten Seite (22) des saugfähigen Hygieneartikels (20) gesichert ist.

## Revendications

1. Dispositif de surveillance d'hygiène (1), comprenant un panneau de détection flexible allongé (2) définissant un axe longitudinal (Xₛ) et un axe transversal (Ys) et étant plus long dans la direction longitudinale qu'il n'est large dans la direction transversale, et une unité d'enregistreur de données (3) fixée ou pouvant être fixée de manière amovible au panneau de détection flexible (2), ladite unité d'enregistreur de données (3) définissant un axe longitudinal (X_{d}) et un axe transversal (Y_{d}), et présentant une longueur longitudinale (I_{d}) qui est plus longue que sa largeur transversale (w_{d}), et lorsque l'unité d'enregistreur de données (3) est fixée au panneau de détection (2), l'axe longitudinal (Xₛ) du panneau de détection flexible (2) est aligné le long de l'axe longitudinal (X_{d}) de l'unité d'enregistreur de données, dans lequel le panneau de détection flexible (2) comprend un substrat flexible (4) qui porte au moins un premier élément de détection (5), dans lequel un matériau de fixation (6) est disposé sur une première surface (7) du panneau de détection flexible (2) et dans lequel ladite unité d'enregistreur de données (3) est disposée sur la seconde surface opposée (8) du panneau de détection flexible (2), ledit dispositif (1) étant adapté pour être fixé de manière amovible à un côté faisant face à un vêtement (22) d'un produit d'hygiène absorbant (20), au moyen dudit matériau de fixation (6), ledit produit d'hygiène absorbant (20) définissant un côté faisant face au corps (21), destiné à faire face au corps du porteur en utilisation, et un côté faisant face à un vêtement opposé (22), destiné à faire face à l'opposé du porteur en utilisation,
dans lequel ledit substrat flexible (4) est une carte de circuit imprimé flexible, et ledit au moins premier élément de détection (5) est connecté électriquement par des fils conducteurs à un moyen de fixation d'enregistreur côté panneau (13),
dans lequel le rapport de la largeur transversale (wₛ), dans l'étendue longitudinale (14) du panneau de détection flexible (2) couverte par l'unité d'enregistreur de données (3), à la longueur longitudinale du panneau de détection est de 0,025 à 0,1,
dans lequel la largeur transversale (ws) du panneau de détection flexible (2), dans l'étendue longitudinale (14) du panneau de détection flexible (2) couverte par l'unité d'enregistreur de données (3), est de 20 à 30 mm, et dans lequel la longueur longitudinale (Is) du panneau de détection flexible (2) est de 200 à 700 mm.

2. Dispositif selon la revendication 1, dans lequel la largeur transversale (w_{d}) de l'unité d'enregistreur de données (4) n'est pas supérieure à 160 % de la largeur transversale maximale (wₛ) du panneau de détection flexible dans l'étendue longitudinale (14) du panneau de détection flexible (2) couverte par l'unité d'enregistreur de données (3) et/ou dans lequel la largeur transversale (wd) de l'unité d'enregistreur de données (3) est d'au moins 80 % de la largeur transversale (wₛ) du panneau de détection flexible (2) dans l'étendue longitudinale (14) du panneau de détection flexible (2) couverte par l'unité d'enregistreur de données (3).

3. Dispositif selon l'une quelconque des revendications précédentes, dans lequel le panneau de détection flexible (2), sur au moins 60 % de son extension longitudinale, présente des bords latéraux s'étendant longitudinalement sensiblement droits et parallèles.

4. Dispositif selon l'une quelconque des revendications précédentes, dans lequel le rapport largeur transversale (w_{d}) / longueur longitudinale (I_{d}) de l'unité d'enregistreur de données est compris entre 0,3 et 0,8

5. Dispositif selon l'une quelconque des revendications précédentes, dans lequel la largeur transversale (w_{d}) de l'unité d'enregistreur de données (3) est de 20 à 45 mm, et/ou dans lequel la longueur longitudinale (I_{d}) de l'unité d'enregistreur de données (3) est comprise entre 30 et 70 mm.

6. Dispositif selon l'une quelconque des revendications précédentes, dans lequel l'unité d'enregistreur de données (3) peut être fixée de manière amovible au panneau de détection flexible (2), et l'épaisseur de l'unité d'enregistreur de données, dans la direction orthogonale à l'axe longitudinal (Xd) et à l'axe transversal (Yd) de l'unité d'enregistreur de données (3), est de 2 à 20 mm.

7. Dispositif selon l'une quelconque des revendications précédentes, dans lequel le produit d'hygiène absorbant (20) est une couche, telle qu'une couche de type culotte, une couche de type ouverte, une couche de type ceinture, ou une serviette pour incontinence ou une serviette hygiénique.

8. Dispositif selon l'une quelconque des revendications précédentes, adapté pour détecter la présence de fluides corporels absorbés par le produit d'hygiène absorbant.

9. Dispositif selon l'une quelconque des revendications précédentes, dans lequel l'unité d'enregistreur de données (3), lorsqu'elle est fixée au panneau de détection (2), est connectée électriquement au au moins un élément de détection (5), de préférence dans lequel ledit élément de détection (5) comprend une première plaque de détection (51) et une seconde plaque de détection (52), et le dispositif est configuré pour mesurer l'impédance entre lesdites première et seconde plaques de détection.

10. Dispositif selon l'une quelconque des revendications précédentes, dans lequel le panneau de détection flexible (2) s'étend longitudinalement au-delà des deux bords longitudinaux de l'unité d'enregistreur de données (3).

11. Dispositif selon l'une quelconque des revendications précédentes, dans lequel la distance entre le bord longitudinalement le plus extérieur de l'unité d'enregistreur de données (3) et le plus proche des bords longitudinalement les plus extérieurs du panneau de détection flexible (2) est d'au moins 10 mm.

12. Dispositif selon l'une quelconque des revendications précédentes, dans lequel un moyen de fixation d'enregistreur côté panneau (13) est disposé sur la seconde surface (8) sur le panneau de détection flexible (2), un moyen de fixation d'enregistreur côté enregistreur (34) étant agencé sur l'unité d'enregistreur de données (3), et dans lequel le moyen de fixation d'enregistreur côté panneau (13) et le moyen de fixation d'enregistreur côté enregistreur (34) sont adaptés pour fixer de manière amovible l'unité d'enregistreur de données (3) au panneau de détection flexible (2), et pour connecter électriquement l'unité d'enregistreur de données (2) au au moins un élément de détection (5).

13. Système comprenant un dispositif de surveillance d'hygiène (1) tel que défini dans l'une quelconque des revendications 1 à 12 et au moins un article d'hygiène absorbant (20), de préférence dans lequel ledit dispositif de surveillance d'hygiène (1) est fixé de manière amovible sur un côté faisant face à un vêtement (22) de l'article d'hygiène absorbant (20).
